# EUROPÄISCHE PATENTSCHRIFT

(11) **EP 3 461 750 B1**
(45) Veröffentlichungstag und Bekanntmachung des Hinweises auf die Patenterteilung: **06.03.2024**
(21) Anmeldenummer: 18206828.8
(22) Anmeldetag: 05.01.2015
(51) Int. Cl.: B65B 31/04, A61L 2/18, A61L 2/22, B65B 31/08, B65B 55/10, B65D 81/24, B65D 85/62, B65D 65/00, D21C 5/02, B65D 65/42, B65B 55/04, D21H 27/10, B65D 75/00

(54) **BEHÄLTNIS ZUR AUFNAHME VON VERPACKUNGSMATERIAL**
CONTAINER FOR PACKING MATERIAL
RÉCIPIENT DESTINÉ À LA RÉCEPTION DU MATÉRIAU D'EMBALLAGE

(30) Priorität: 09.01.2014 DE 102014100203
(43) Veröffentlichungstag der Anmeldung: 03.04.2019
(62) Teilanmeldung aus: 17176407.9
(73) Patentinhaber: SIG Services AG, 8212 Neuhausen am Rheinfall (CH)
(72) Erfinder: Mainz, Hans Willi, 52525 Heinsberg (DE)
(74) Vertreter: Cohausz & Florack

(56) Entgegenhaltungen:
- DE-A1- 19 748 022
- DE-B3-102011 111 523
- JP-A- 2000 326 935
- US-A- 4 061 785
- US-A- 4 061 785
- US-A1- 2012 005 948
- US-A1- 2013 065 958
- US-A1- 2013 065 958

## Beschreibung

Die Erfindung betrifft Behältnisse zur Aufnahme von Verpackungsmaterial, wobei wenigstens Teile der Innenseite des Behältnisses mit einem Behandlungsmittel benetzt oder ausgestattet sind und/oder im Inneren des Behältnisses mit einem Behandlungsmittel versehene Beladungsträger vorgesehen sind und dass das Behandlungsmittel eine H₂O₂-Lösung als Entkeimungsmittel enthält.

Aus der US 2013/0065958 A1 ist ein gattungsgemäßes Behältnis bekannt, welches geeignet für die Aufnahme einer Mehrzahl flachgefalteter Packungsmäntel, Zuschnitte oder zu einer Rolle aufgerollter Bahnware ist. Dort wird ein Behandlungsmittel eingesetzt, das eine H₂O₂-Lösung ("peroxide") als Entkeimungsmittel enthält, um die im Behältnis verpackten Gegenstände zu entkeimen. Aus der US 4 061 785 ist es bereits für sich bekannt, ein Behandlungsmittel zu verwenden, das aus einer Kombination von Ethanol und einem Konservierungsmittel besteht. Die dort beschriebenen Behandlungsmittel werden zur Erhöhung der Haltbarkeit von Obst, Gemüsen, Blumen oder dergleichen eingesetzt. Eine Entkeimung von Verpackungsmaterial wird darin nicht beschrieben.

Die Herstellung von Karton/Kunststoff-Verbundpackungen erfolgt entweder als sog. "Schlauchformungsverfahren" von der Rolle oder aus einzelnen Zuschnitten aus Papier/Kunststoff-Laminatmaterial. Hier werden zunächst aus einer Rolle Verbundmaterial einzelne Zuschnitte gewonnen und diese anschließend mit einer dichten Längsnaht versehen, die im Allgemeinen durch Faltung und Versiegeln des Verbundmaterials und ggf. durch zusätzliches Überkleben mit einem Dichtstreifen erzeugt wird.

Die Behandlung von offenen Schnittkanten eines Packungsmantels, Zuschnitts oder einer zu einer Rolle aufgerollten Bahnware eines Verpackungsmaterials, insbesondere Karton/Kunststoff-Verbundmaterials, erfolgt durch Aufbringen bzw. Einbringen eines ein Entkeimungsmittel aufweisenden Behandlungsmittels auf bzw. in den äußeren Bereich der Schnittkanten.

Wenn im Folgenden von offenen Schnittkanten' die Rede ist, so sollen darunter nicht nur die Schnittkanten verstanden werden, die einen Zuschnitt oder einem daraus geformten Packungsmantel begrenzen, sondern sämtliche "offenen" also der umgebenden Atmosphäre zugänglichen Zellstoffbereiche, so dass insbesondere auch Schnittkanten im Inneren von Perforationen gemeint sind, wie sie bei Getränkepackungen im Bereich von aufzubringenden Ausgießelementen oder anderer Öffnungshilfen anzutreffen sind. Die offenen Schnittkanten bilden Zellstoffbereiche, die der umgebenden Atmosphäre zugänglich sind.

Die weitere Verarbeitung derart hergestellter Packungsmäntel, also das einseitige Verschließen auf der Ober- oder Unterseite der späteren Verpackung, das Entkeimen, das Befüllen und erneute Verschließen erfolgt meist direkt in der Füllmaschine.

Hier erfolgen dann Becherformung, Reinigung und gegebenenfalls Desinfektion bevor das Füllgut eingefüllt und die Verbundverpackung verschlossen und endgültig ausgeformt wird. Desinfektion und Befüllung erfolgen bei einer Herstellung einer so genannten aseptischen Verbundverpackung in der aseptischen Zone einer Füllmaschine. Erfolgt die Becherformung vor der Desinfektion beziehungsweise der Sterilisation, kann die Becherformung auch außerhalb der Aseptikzone stattfinden. Derartige Verfahren sind unter anderem in der DE 32 35 476 A1 und der DE 10 2009 029 706 A1 beschrieben.

Unabhängig vom Herstellungsverfahren erfolgt das Verschließen der Packung in der Regel durch Zusammenpressen und Versiegeln der Packstoffkanten beispielsweise durch Ultraschall mittels einer Sonotrode und einem Amboss. Es sind auch andere Verfahren zum Verschließen der Packung bekannt, beispielsweise elektromagnetische Induktion oder die Baufschlagung mit Heißluft in Verbindung mit mechanischem Verpressen.

Es hat sich herausgestellt, dass insbesondere Staub für die Kontamination mit Keimen verantwortlich ist, so dass bei allen Schritten die Vermeidung von Staub im Vordergrund stehen sollte. Dies kann durch Staubabsaugung während der Packungsmantelfertigung und Verringerung der Standzeiten der verwendeten Schneidmesser erfolgen. Bedingt durch die Fasern im Zellstoff des verwendeten Kartons bleiben jedoch die offenen Schnittkanten stets die,Problembereiche' bei der Packungsfertigung. Leistungsfähige Absauganlagen schaffen hier in den meisten Fällen zwar Abhilfe, belasten den Produktionsprozess jedoch durch hohe Energiekosten und Geräuschemissionen.

Unter einer aseptischen Verpackung ist eine Verpackung zu verstehen, in die ein Füllgut, insbesondere ein Lebensmittel, unter aseptischen Bedingungen gefüllt wird. Dazu verwendete Füllmaschinen umfassen eine Aseptikzone, eine Art Reinraum, in dem sterile, also keimfreie Bedingungen herrschen, zu deren Aufrechterhaltung der Raum meist bis auf wenige Öffnungen verschlossen oder weitgehend verschlossen ausgebildet ist. Die darin gebildete Reinraumatmosphäre steht zudem durch Einfuhr von Sterilluft unter Überdruck, so dass keine Keime von außen eindringen können. Das Verpackungsmaterial wird dann kontinuierlich oder diskontinuierlich durch die Aseptikzone transportiert, wobei es in aufeinanderfolgenden Schritten sterilisiert, getrocknet, in einem oder mehreren Schritten gefüllt und verschlossen wird. Pro Bearbeitungsschritt lassen heutige Maschinen nach Stand der Technik, etwa eine Maschine aus der 24er-Baureihe der Anmelderin, je nach Packungsformat eine Bearbeitungszeit einer einzelnen Sterilisations- oder Füllstation von etwa 0,6 Sekunden bis etwa 0,85 Sekunden zu.

Bei den Kanten von Packungsmänteln handelt es sich um offene Schnittkanten eines ansonsten wasserdichten und ggf. auch eine Sauerstoffbarriere aufweisenden Laminatmaterials. Daher besteht die Gefahr des Austausches von Keimen (Mikroorganismen und Sporen) in erster Linie im Bereich der Kanten, wodurch eine Erhöhung der Keimbelastung des Materials erfolgen und schließlich auch eine Kontamination des Produktes beim sich anschließenden Füllen der Verpackung bzw. Verschließen zur fertigen Packung nicht ausgeschlossen werden kann.

Das Verschließen eines einseitig offenen, gefüllten Behälters aus Verbundmaterial birgt das Risiko, dass, insbesondere beim Versiegeln mit Ultraschall, aus den offenen Schnittkanten Staub aus dem Verpackungsmaterial geschleudert werden kann und dieser sowohl den aseptischen Bereich der Füllmaschine als auch die offenen Verpackungen selbst verunreinigen kann.

Als vegetative Mikroorganismen werden durch Zellteilung vermehrungsfähige Einzeller verstanden, die geeignet sind sich im Füllgut ('Produkt'] einer Packung zu vermehren und dabei die Eigenschaften des Füllgutes zu verändern. Weiter umfasst der Begriff auch die Überdauerungsformen der vermehrungsfähigen Einzeller, wie zum Beispiel deren Sporen.

Diese Sporen sind meist sehr resistent gegen Veränderungen der sie umgebenden Umweltbedingungen. Wenn Mikroorganismen keine Umgebung zum Stoffwechsel und/oder zur Fortpflanzung vorfinden, haben einige Mikroorganismen die Möglichkeit, in ein Sporenstadium überzugehen.

Genauer sollen unter dem Begriff Mikroorganismen im Sinne der vorliegenden Anmeldung Eukaryoten und Prokaryoten verstanden werden, wobei die Eukaryoten eine echte Zellwand aufweisen und Algen, Protozonen, Pilze und Schleimpilze umfassen, während die Prokaryoten die Gruppe der Bakterien abdecken (vgl. 'Bergey's Manual of Determinative Bacteriology', 8. Auflage, Baltimore: Williams & Wilkins, 1974).

Speziell bei den Prokaryoten sind Überdauerungsformen, wie beispielsweise Sporen, bekannt. Diese sind vermehrt beispielsweise auch nach thermischen und/oder chemischen Behandlungen von Rohstoffen zur Herstellung von Kartonroherzeugnissen in eben diesen anzutreffen, da derartige Behandlungsmethoden die direkt vermehrungsfähige Form der Mikroorganismen entweder abtötet oder den Übergang in die Sporenform initiiert.

Als Maß für die Anzahl beziehungsweise die Menge der in einer Stoffmenge, (beispielsweise in dem angesprochenen Kartonroherzeugnis) enthaltenen Mikroorganismen, ist dem Fachmann der Begriff der "koloniebildenden Einheit pro Gramm" (kbE/g) bekannt. Im Unterschied zu der direkten Auszählung aller vorhandenen Mikroorganismen mit einem geeigneten optischen Mittel, erfolgt die Bestimmung der Anzahl der koloniebildenden Einheiten über die gezielte Vermehrung der vorhandenen teilungsfähigen Mikroorganismen unter geeigneten Anzuchtbedingungen. Im Allgemeinen geschieht dies bis zu einer Koloniegröße, die mit dem unbewehrten Auge zählbar ist. Dabei nutzt man die Tatsache, dass aus jedem einzelnen teilungsfähigen Mikroorganismus unter zuvor definierten Bedingungen genau eine Kolonie entsteht. Einzelfälle, in denen zwei kbE so nah zusammenliegen, dass sich nur eine sichtbare Kolonie aus ihnen bildet, werden dann regelmäßig vernachlässigt.

In der Mikrobiologie typische Bestimmungsverfahren sind in der ISO 8784-1 aus 2005 geregelt.

Eine Reduzierung der kbE/g wird vom Fachmann demzufolge als Maß für die Wirksamkeit eines Verfahrens zur Keimreduktion verwendet und häufig Entkeimungsrate genannt. Daraus abgeleitet ergibt sich die über die Anzahl von produzierten Packungen zu zählende Sterilitätsrate.

Es ist aus der DE 10 2011 111 523 A1 bereits bekannt, oben bzw. unten offene Schnittkanten eines Packungsmantels eines Verpackungsmaterials durch Aufbringen eines Behandlungsmittels, welches ein Entkeimungsmittel enthält, zu behandeln, wobei das Entkeimungsmittel nach dem Aufbringen auf den Schnittkanten verbleibt und in das Verpackungsmaterial eindringt. Das Aufbringen geschieht dabei jeweils durch Besprühen von oben, wobei eine Mehrzahl von Packungsmänteln flachgefaltet zusammengefasst sind. Bei diesem bekannten Verfahren erfolgt das Aufbringen des Behandlungsmittels auf die Packungsmantelkanten in einer eigens dafür konstruierten Station in einem oder mehreren separaten Verfahrensschritten unmittelbar vor dem Verpacken der Packungsmäntel in einen Umkarton. Diese Vorgehensweise ist relativ aufwändig. Auch besteht die Gefahr, dass versehentlich an der Packung vorbei gesprüht wird. Zudem hat sich gezeigt, dass die Außenseiten der Packungsmäntel rasch in Mitleidenschaft gezogen werden können. Beispielsweise kann das Behandlungsmittel in ungewollter Weise auf das Druckbild auf der Außenseite des Packungsmantels einwirken und dieses beschädigen. Auch kann es vorkommen, dass zwei Packungsmäntel nach Entnahme aus dem Umkarton aneinander kleben bleiben und bei ihrer weiteren Verarbeitung in der Füllmaschine zu Produktionsstörungen führen können.

Zum Aufbringen des Behandlungsmittels wird wenigstens ein Teil des Behandlungsmittels nicht unmittelbar auf die Schnittkanten aufgebracht, sondern eine Beladung eines Behandlungsträgers erfolgt, welcher das Behandlungsmittel aufnimmt, so dass eine sich einstellende aktiv sterilisierende Atmosphäre anschließend auf die offenen Schnittkanten des Packungsmantels, Zuschnitts oder der Bahnware einwirkt und die gewünschte Sterilisation vornimmt.

Dabei kann eine Sterilisation bis in eine ausreichende Tiefe in den Karton hinein erreicht werden, ohne dass der bekannte und bewährte Ablauf der Packungsfertigung beeinflusst werden soll. Die "ausreichende Tiefe" wird dabei durch das jeweils verwendete Herstellungsverfahren bestimmt. Erfolgt die Nahtbildung mittels Ultraschall, so muss eine Sterilisation bis etwa 2 bis 3 mm Tiefe erfolgen, um zuverlässig auszuschließen, dass beim Siegelvorgang kontaminierter Staub aus der offenen Kante geschleudert wird. Sollte die Naht mittels Mikrowellentechnologie geschlossen werden, reicht auch eine Tiefe von wenigen Zehntel Millimeter aus.

Davon ausgehend liegt der Erfindung die Aufgabe zugrunde, ein Behältnis zur Aufnahme einer Mehrzahl flachgefalteter Packungsmäntel, Zuschnitte oder zu einer Rolle aufgerollter Bahnware bereit zu stellen, in dem das vorgenannte Verpackungsmaterial mit sterilisierten offenen Schnittkanten gelagert oder transportiert wird.

Gelöst wird diese Aufgabe bei einem Behältnis zur Aufnahme von Verpackungsmaterial mit den Merkmalen des Oberbegriffs von Patentanspruch 1 dadurch, dass das Verpackungsmaterial aus einem Verbundwerkstoff mit wenigstens einer Lage Karton, welcher Zellstoff enthält, besteht, dass das Behältnis Verpackungsmaterial in Form von einer Mehrzahl flachgefalteter Packungsmäntel, Zuschnitte oder zu einer Rolle aufgerollter Bahnware enthält, jeweils mit offenen Schnittkanten, wobei die offenen Schnittkanten der umgebenden Atmosphäre zugängliche Zellstoffbereich bilden und dass das Behandlungsmittel Ethanol enthält..

Bei einem Verfahren zum Verpacken von einer Mehrzahl flachgefalteter Packungsmäntel, Zuschnitte oder zu einer Rolle aufgerollter Banware, jeweils aus einem Verbundwerkstoff mit wenigstens einer Lage Karton, welcher Zellstoff enthält, und mit offenen Schnittkanten, in einem solchen Behältnis erfolgt die Lösung der Aufgabe durch die folgenden Schritte:
- Einbringen der flachgefalteten Packungsmäntel, Zuschnitte oder zu einer Rolle aufgerollter Bahnware, jeweils mit offenen Schnittkanten, in das Behältnis,
- Aufbringen eines Ethanol und eine H₂O₂-Lösung als Entkeimungsmittel enthaltendes Behandlungsmittels auf die Innenseite des Behältnisses und/oder auf einen Beladungsträger und Einbringen des Beladungsträgers in das Behältnis und
- Verschließen des Behältnisses.

Die Übertragung des Behandlungsmittels erfolgt indirekt durch Verdampfung und anschließende Absorption in der/den offenen Schnittkante/n des Verpackungsmaterials.

Des Weiteren können bevorzugt die äußeren Oberflächen des Behältnisses wenigstens im perforierten Bereich versiegelt sein, um eine Migration des Behandlungsmittels an die Umgebung zuverlässig zu verhindern. pe Behältnisse werden weiter unten anhand der Ausführungsbeispiele näher erläutert.

Nach einer weiteren Lehre der Erfindung wird als Material für das Behältnis Karton, insbesondere Wellpappe, verwendet, so dass ein Umkarton mit schließbaren Laschen entsteht.

Handelt es sich bei dem Behältnis um einen Umkarton aus Wellpappe, bietet das den Vorteil, dass die Absorption des Sterilisationsmittels aus den beladenen Umkartonlaschen durch die oberen oder unteren offenen Mantelkartonkanten der verpackten Mäntel im Umkarton stattfindet. Die im Gegensatz zu einer Sterilisation von Packungsmäntel nach der DE 10 2011 111 523 A1 ohnehin schon deutlich reduzierte Arbeitsbelastung kann dabei noch einmal verringert werden, wenn der Umkarton außerhalb der direkten Produktionslinie, etwa in einem benachbarten Raum mit dem Behandlungsmittel beaufschlagt wird. Dabei kann es fallweise sogar besonders bevorzugt sein, wenn der seinerseits als Faltschachtel vorliegende Umkarton bereits vor oder während des Auffaltens des Umkartons beladen wird.

Als Material für das Behältnis kann in weiterer Ausgestaltung der Erfindung auch Kunststoff verwendet werden. Dabei kann das Behältnis nach einer bevorzugten Lehre der Erfindung einen Kunststoffcontainer und einen Kunststoffdeckel sowie wenigstens einen Beladungsträger aufweisen. Alternativ ist es auch möglich, dass das Behältnis eine Schrumpffolie mit einem Beladungsträger ist.

Eine "Impfung" der vom Behältnis, vorzugsweise der vom Umkarton umschlossenen Atmosphäre nach Verschließen des Kartons ist beispielsweise mit einer Nadel möglich, wobei der Umkarton an geeigneten Stellen, beispielsweise am freien Kopfraum ein- oder mehrfach, durchstochen werden muss. Es ist zudem denkbar, dass ein Beladungsträger, beispielsweise ein Filzstreifen, vor dem Verschließen auf die Innenseite des Umkartons appliziert wird und nach dem Verschließen des Umkartons mit einer Nadel so durchdrungen wird, dass die Nadel das Behandlungsmittel in den Beladungsträger injizieren kann. Auf diese Weise wird die Umgebung praktisch nicht durch die in dem Behandlungsmittel enthaltenen Wirkstoffe belastet.

Dazu kann das Behältnis auch ein Ventil umfassen. Durch die Verwendung eines Ventils wird ein Wiederaustreten des Behandlungsmittels aus dem Behältnis beziehungsweise ein übermäßiges Diffundieren wirksam unterbunden. Das Ventil kann als mechanisches Einwegeventil oder als durchstoßbare und selbstschließende Membran ausgebildet sein. Das Vorsehen eines mechanischen Ventils kann insbesondere bei wiederverwendbaren Behältnissen von Vorteil sein, während eine selbstschließende Membran auch ganz einfach von außen oder innen an das Behältnis geklebt werden kann und wenigstens für einen einmaligen Gebrauch ausreichend Schutz bietet.

Bevorzugt ist das Behandlungsmittel mit einem geeigneten Farbstoff eingefärbt. Dadurch lässt sich bereits optisch der Anbringungsbereich leicht feststellen. Dies kann zu Kontrollzwecken dienen. Wird darüber hinaus ein Farbstoff verwendet, der gemeinsam mit dem Entkeimungsmittel aus der aktiv sterilisierenden Atmosphäre von den offenen Schnittkanten des Verpackungsmaterials absorbiert wird, ist eine erfolgte Behandlung auch am Verpackungsmaterial selbst durch eine einfache Sichtkontrolle nachprüfbar. Dabei kann dann auch die Eindringtiefe des Behandlungsmittels in den Randbereich erkannt werden. Es ist also bevorzugt, dass der Farbstoff eine Verfärbung der behandelten Kanten bewirkt.

Eine Beladung einer Umverpackung an geeigneter Stelle, beispielsweise der Innenseiten der Laschen eines Umkartons, mit einem ein Entkeimungsmittel beinhaltenden Behandlungsmittels, beispielsweise einer Peroxid-Lösung, kann durch Sprühen, Tropfen und anschließendes Verstreichen, Streichen, Rollen, Tupfen, Spateln, Bedrucken (beispielsweise mit einem "Inkjet"-Verfahren), Tauchen oder Bedampfen erfolgen. Auch kann die Beladung - wie bereits erwähnt - als Injektion vorgenommen werden.

Das Beaufschlagen der Kanten der in einem Behältnis zu transportierenden Verpackungsmaterialien mit Entkeimungsmittel kann auch durch Anbringen eines mit einem Sterilisationsmittel beladenen Streifens, Einlageblattes oder anderen Trägermaterials erfolgen.

Dabei kann es von Vorteil sein, wenn der Beladungsträger als Akkumulator ausgebildet ist und durch flüssiges Behandlungsmittel wieder aufgefüllt werden kann, um das Behandlungsmittel dann wieder an die Umgebung abzugeben. Geignet sind dafür insbesondere porige beziehungsweise zellige Speichermaterialien.

Die in einem Behältnis, insbesondere einem Umkarton verpackten flachgefalteten Verpackungsmaterialien werden in der Regel über einen Zeitraum von mehreren Tagen transportiert. Währenddessen findet eine Keimreduktion bzw. Sterilisation der offenen Kartonkanten und des Behältnisses bzw. der Umverpackung durch das absorbierte Sterilisationsmittel statt. Dabei hat man überraschender Weise festgestellt, dass es trotz sehr geringer Konzentrationen des Entkeimungsmittels zu erstklassigen Entkeimungsraten kommt. Es wird nun davon ausgegangen, dass die geringen Konzentrationen des Entkeimungsmittels die lange Verweilzeit im Behältnis als wirksame Einwirkzeit nutzen kann. Die sich einstellende aktiv sterilisierende Atmosphäre kann also über einen langen Zeitraum von mehreren Stunden oder sogar Tagen in einem wirksamen Rahmen aufrechterhalten werden. Es kommt also zu einer langen Einwirkzeit bei geringer Konzentration. Dabei dringt das Entkeimungsmittel - je nach verwendeter Menge - bis zu 2 mm oder 3 mm oder gar noch tiefer in den Randbereich bzw. Perforationsbereich des Kartons ein. Dies ist wichtig, weil die dort in der Zellstoff- /Kartonlage angesiedelten Fasern als nicht ortsfest zu bezeichnen sind und damit auch die auf den Fasern angesiedelten Keime nicht gebunden sind sondern mit den Fasern als Staub aus der Zellstoff-/Kartonlage austreten können.

Die Einwirkzeit ist, jeweils bezogen auf eine bestimmte Kartonsorte und -grammatur, abhängig vom verwendeten Behandlungsmittel bzw. den enthaltenen Beigaben, der Dosierung, der erwarteten Entkeimungsrate und der Umgebungstemperatur.

Eine Absorption von Behandlungsmittel durch die Kartonkante ist möglich, da der für die herzustellenden Packungen eingesetzte Karton hydrophil ist, und im allgemeinen als Fertigprodukt eine relative Feuchte von etwa 5,5 % - 8,5 % aufweist, d.h. die Feuchte des Kartons liegt weit unterhalb der Sättigungsgrenze, die sich aus den herrschenden Umweltbedingungen ergibt und allgemein mit der Gleichgewichtsfeuchte zur Umgebung identisch ist.

Der Zusatz eines polaren organischen Lösungsmittels, das einen geringeren Dampfdruck als das Wirkmittel, hier also das Entkeimungsmittel, aufweist, wirkt dabei als Beschleuniger. So begünstigt der Zusatz an Ethanol zu der Peroxid-Lösung die Absorptionswirkung der Schnittkante und erhöht die Penetration des Peroxids in den Karton. Dadurch wird ein tieferes Eindringen des Entkeimungsmittels in die Schnittkanten ermöglicht, so dass bei geeignetem Verhältnis von Entkeimungsmittel und Ethanol bereits nach wenigen Stunden ein Randbereich des zu behandelnden Verpackungsmaterials von bis zu mehreren Millimetern Tiefe sterilisiert sein kann.

Ethanol gehört zu der Gruppe der polaren organischen Lösungsmittel, die allgemein als Beschleuniger wirken, wenn ihr Dampfdruck geringer ist als der des Wirkmediums. Ist der Dampfdruck des beigemischten polaren organischen Lösungsmittels höher als der des Wirkmittels, wirkt das Lösungsmittel als Verzögerer. Ein Beispiel eines Lösungsmittels mit einem höheren Dampfdruck ist DOWANOL^{®}, ein Markenprodukt der DOW Chemical Company. Allgemein kann man sagen, dass niedermolekulare polare organische Lösungsmittel einen niedrigen Dampfdruck aufweisen und höhermolekulare organische Lösungsmittel einen hohen Dampfdruck aufweisen.

Zusätzlich wird die Sterilisationswirkung von einigen Sterilisationsmitteln synergetisch verstärkt.

### Bestimmungsmethode:

Zur näheren Definition der in den Ansprüchen genannten Merkmale wird folgende Bestimmungsmethode festgelegt:

### Bestimmung der koloniebildenden Einheiten je Gramm (kbE/g)

Die europäische Standardmethode ISO 8784-1:2005 und die in dieser Prüfvorschrift angeführten Referenzen werden als Grundlage für die Bestimmung der koloniebildenen Einheiten je Gramm(kbE/g) gewählt. Die Prüfvorschriften werden hier für die Untersuchung der kbE/g-Belastung von Verpackungs- bzw. Verbundmaterial aus Karton, Kunststoff und teilweise Aluminium angewendet. Sollten in der Durchführung der Probennahme und Bestimmung der kbE/g des Verpackungsmaterials Abweichungen von den zitierten Prüfvorschriften notwendig sein, so werden sie im Folgenden erläutert.

### 1.1.1. Probennahme und Probenvorbereitung

Die entnommenen Verpackungsmaterialproben dürfen nicht mit den Händen angefasst werden. Die Aufbewahrung geschieht in sterilen Probenahmegefäßen, vorzugsweise sterilen Plastikbeuteln. Die zu untersuchenden Bereiche werden mit einer sterilen Schere in Stücke geschnitten.

"Zu untersuchende Bereich" sind:
a. Der erste Teilbereich, der kein Randbereich ist (Probennahme min. 10 Millimeter von der offenen Schnittkante entfernt)
b. Der zweite Teilbereich bzw. Randbereich, an den offenen Schnittkanten bzw. den Perforationskanten des Verpackungsmaterials (bis max. drei Millimeter ab der offenen Schnittkante)

In Ergänzung zur europäische Standardmethode ISO 8784-1:2005, Kapitel 8 "Preparation of the test material", werden für die ersten Teilbereiche max. 3 g Verpackungsmaterial eingesetzt. Für den zweiten Teilbereich werden ebenfalls max. 3 g Verpackungsmaterial eingesetzt. Sollte aus einem Probenstück nicht ausreichend Material erhalten werden, so werden die zweiten Teilbereiche aus max. 5 gleichen Probenstücken zusammen eingesetzt.

### 1.2. Optionale Bestimmung der Oberflächenkeimzahl

Es besteht die Möglichkeit, dass die Verpackungsmaterialproben vor der Probenahme zusätzlich zu dem Vorhandensein von Keimen im Innern auch oberflächlich verkeimt sind. Einen Fehler der Bestimmung der kbE/g des Verpackungsmaterials kann dadurch verhindert werden, dass die Oberflächenkeimzahl separat bestimmt wird und die Oberflächenkeimzahl von dem unter 1.1. bestimmten Zahlwert der koloniebildenden Einheiten je Gramm subtrahiert wird.

### 1.2.1. Begriffe und Abkürzungen

ml = Milliliter
h = Stunde
kbE = Kolonie bildende Einheiten
°C = Grad Celsius
g = Gramm
mm = Millimeter
cm = Zentimeter

### 1.2.2. Benötigte Hilfsmittel

Kontakt-Petrischalen (Kunststoff) ∅ 5,5 cm (z.B. Greiner Bio-one 629180) Pinzetten
Cutter-Messer
Steriler Folienbeutel oder Aluminiumfolie
Sterilisator
Brutschrank
sterile Werkbank
Nährmedien: Plate-Count-Agar (z.B. erhältlich als Oxoid Nr. CM 325, Merck Nr. 105463, Difco Nr. 247940)

### 1.2.3. Durchführung

Es werden pro Probe 240 cm² Verpackungsmaterialfläche untersucht.

Eine Kontakt-Petrischalen hat eine Fläche 24 cm². Daher sind 10 Kontaktschalen pro Probe vorzubereiten, um die o.g. Fläche zu untersuchen.

In die sterilen Petrischalen wird so viel Nährboden gegeben, dass der Agar über die Petrischalenkante hinausragt (Wölbung), jedoch nicht über den Rand hinausläuft. Die vorbereiteten erkalteten Kontaktschalen werden auf die Oberfläche des Verpackungsmaterials gedrückt, verschlossen und bei den angegebenen Bedingungen inkubiert.

Es ist darauf zu achten, dass die Probestücke nur mit einer sterilen Pinzette berührt werden und die Kontaktschalen nicht mit der offenen Packungskante in Berührung kommen.

Die Petrischalen werden mit dem Deckel nach unten in den Brutschrank gelegt, um Kondenswasserbildung zu vermeiden.

Nährboden: Für die Oberflächenkeimzahl wird Plate-Count-Agar verwendet.

Bebrütung:_Die Bebrütung des Plate-Count-Agars erfolgt 5 Tage bei 30 °C, wobei die Auswertung im Anschluss daran stattfindet.

### 1.2.4. Auswertung

Gezählt werden alle vorhandenen Kolonien auf den Kontaktplatten einer Probe. Die Ergebnisse werden auf kbE (koloniebildende Einheiten) / 100 cm² umgerechnet und dokumentiert. Der erhaltene Wert wird auf die Oberfläche der zu untersuchenden Bereiche umgerechnet und als Zahlenwert von der dem Wert der koloniebildenden Einheiten je Gramm subtrahiert.

### Beispiele:

Zur praktischen Umsetzung des erfindungsgemäßen Verfahrens kann als Behandlungsmittel beispielsweise eine Wasserstoffperoxidlösung von 3,5 % bis 50 % mit oder ohne Zusatz von
- Verdampfungsverzögerer (beispielsweise DOWANOL^{®})
- Mitteln die die Oberflächenspannung reduzieren, wie beispielsweise Alkoholanteile (Alkoholanteile mit Verzögerungsmittel, oberflächenaktive Substanzen wie Tenside etc.)
- Peressigsäure 3-15 %
- n- oder Isopropanol
- anderen sporizid wirkende Mitteln
verwendet werden.

Die Lösung kann in Mischung mit Wasser oder Alkohol (Ethanol) verarbeitet werden, sie ist dabei ohne toxische Rückstände oder vollständig verflüchtigbar.

In Tabelle 1 sind 12 Anwendungsbeispiele aufgeführt. Es gelten folgende Grundbedingungen für diese Beispiele:
Die Auswertung der kbE/g erfolgte nach der in den Methoden beschriebenen Normen und Verfahren. Das behandelte Verpackungsmaterial hat als Kartonlage einen Karton der Firma Stora Enso Natura Board mit einer Grammatur von 230 g/m². Wenn nicht anders beschrieben, wurden 350 Verpackungsmäntel in einem Behältnis aufbewahrt.

Die Behältnisse haben folgende Eigenschaften:
Umkarton: Aus Wellpappe hergestellter Container mit den Maßen B: 60 cm; H: 19 cm; T: 10,5 cm
Kunststoffbox: Aus Polypropylen hergestellter Container mit Deckel mit den Maßen B: 60cm H: 19 cm T: 10,5 cm
Schrumpffolie: Aus Polyolefinen hergestellte Folie mit den Maßen B: 1m; L: 2,5 m; Dicke: 20µm
Beladener Papierträger: DIN A4, Zellstoff mit einer Grammatur von 150g/m²

Das Behandlungsmittel ergibt sich aus folgenden Chemikalien:
H₂0₂-Lösung: Peroxal DS 35% H₂O₂-Lösung (Lebensmittelqualität)
Ethanol: Ethanol technisch (99%) mit MEK vergällt (1%)
Methanol: Methanol technisch (98%)
Dowanol: 1-Methoxy-Propanol-2 von der Firma Dow

**Tabelle 1: Untersuchung verschiedener Anwendungsbeispiele**

| | Behandlungsmittel | Volumenanteil | Auftragsvolumen | Art der Auftragung | Behältnis | Unbehandelt * | 1 h Einwirkzeit* | 48 h Einwirkzeit* |
|---|---|---|---|---|---|---|---|---|
| Beispiel 1 | H2O2-Lösung | 1 | 1ml | Bestreichen der Umkartonlasche | Umkarton | - | - | ++ |
| | Ethanol | 1 | | | | | | |
| | Wasser | 1 | | | | | | |
| Beispiel 2 | H2O2-Lösung | 10 | 10ml | Bestreichen der Umkartonlasche | Umkarton | - | + | +++ |
| | Ethanol | 1 | | | | | | |
| | Wasser | 1 | | | | | | |
| Beispiel 3 | H2O2-Lösung | 1 | 5ml | Bestreichen der Umkartonlasche | Umkarton | - | - | + |
| | Ethanol | 5 | | | | | | |
| | Wasser | 5 | | | | | | |
| Beispiel 4 | H2O2-Lösung | 2 | 2ml | Bestreichen der Umkartonlasche | Umkarton | - | - | +++ |
| | Ethanol | 2 | | | | | | |
| | Wasser | 1 | | | | | | |
| Beispiel 5 | H2O2-Lösung | 2 | 5ml | Besprühen der Umkartonlasche | Umkarton | - | + | +++ |
| | Ethanol | 2 | | | | | | |
| | Wasser | 1 | | | | | | |
| Beispiel 6 | H2O2-Lösung | 1 | 1ml | Besprühen der Umkartonlasche | Umkarton | - | - | ++ |
| | Ethanol | 1 | | | | | | |
| | Wasser | 1 | | | | | | |
| Beispiel 7 | H2O2-Lösung | 2 | 1ml | beladener Papierträger | Kunststoffbox | - | - | +++ |
| | Ethanol | 2 | | | | | | |
| | Wasser | 1 | | | | | | |
| Beispiel 8 | H2O2-Lösung | 2 | 2ml | beladener Papierträger | Umkarton | - | - | ++ |
| | Ethanol | 2 | | | | | | |
| | Wasser | 1 | | | | | | |
| Beispiel 9 | H2O2-Lösung | 2 | 2ml | beladener Papierträger | Schrumpffolie | - | - | +++ |
| | Ethanol | 2 | | | | | | |
| | Wasser | 1 | | | | | | |
| Beispiel 10 | H2O2-Lösung | 1 | 1 ml | Bestreichen der Umkartonlasche | Umkarton | - | - | - |
| | Ethanol | 1 | | | | | | |
| | Wasser | 5 | | | | | | |
| Beispiel 11 | H2O2-Lösung | 1 | 1 ml | Bestreichen der Umkartonlasche | Umkarton | - | 1 - | ++ |
| | Methanol | 1 | | | | | | |
| | Wasser | 1 | | | | | | |
| Beispiel 12 | H2O2-Lösung | 1 | 1 ml | Bestreichen der Umkartonlasche | Umkarton | - | - | - |
| | Dowanol | 1 | | | | | | |
| | Wasser | 1 | | | | | | |
| | * KBEs im definierten Randbereich (bis zu 3 mm) der offenen Schnittkante des Verpackungsmaterials | | | | | | | |

Legende:

| | | | |
|---|---|---|---|
| - | > 1000 kbE/g | + | < 50 kbE/g |
| - | 500 kbE/g < x < 1000 kbE/g | ++ | < 10 kbE/g |
| - | 100 kbE/g < x < 500 kbE/g | +++ | < 1 kbE/g |

Zusammenfassend können die Vorteile der Erfindung unter den folgenden Stichworten beschrieben werden:
- Behandlungsmittel kann definiert auf die Umkartonlaschen aufgebracht werden.
- Minimaler Verbrauch an Entkeimungsmittel im Vergleich zur Direktbesprühung der Kanten. Gefahrenpotenzial und Belastung für die Arbeitsumgebung sind dadurch deutlich verringert.
- Überbeladung durch direktes Aufsprühen wird vermieden.
- Zeit zum Absorptionsaustausch zwischen Kartonlasche/Mantelkante bzw. Perforation ist ausreichend vorhanden durch Transport vom Herstellort zum Abfüllbetrieb (häufig größer 3 Tage).

- Kennzeichnung durch Färben des Behandlungsmittels auf den Umkartonlaschen sinnvoll, daher gute Aufbringungs- und Dosierkontrolle möglich.
- Durch minimale Absorptionsmengen sind keine nachweisbaren Entkeimungsmittelrestmengen, z.B. Peroxidrestmengen vor Verarbeitung des Verpackungsmaterials an der Füllmaschine vorhanden. Eine Gesundheitsgefährdung ist daher ausgeschlossen.
- Keine Beeinflussung des sichtbaren Bereichs der Verpackungsaußenseite. Insbesondere wird das für den Verkauf der Verbundverpackung wichtige Druckbild nicht angegriffen und ein Aneinanderhaften benachbarter Packungsmäntel im Zuführprozess einer Füllmaschine wirksam verhindert.
- Das Behandlungsmittel, insbesondere die Peroxidlösung, wirkt praktisch ausschließlich auf die offenen Schnittkanten, weil es von diesen aktiv absorbiert wird. Keine Belastung der übrigen Packung.

Die Erfindung wird nachfolgend anhand einer lediglich Ausführungsbeispiele darstellenden Zeichnung näher erläutert. In der Zeichnung zeigen
- Fig. 1A: einen Zuschnitt für Getränkekartons in Draufsicht,
- Fig. 1B: einen flachgefalteten Packungsmantel, hergestellt aus einem Zuschnitt gemäß Fig. 1A,
- Fig. 2: einen Umkarton zur Aufnahme einer Vielzahl von Packungsmänteln mit oben offenen Kartonlaschen im Vertikalschnitt,
- Fig. 3: ein erstes Ausführungsbeispiel eines oben offenen Umkartons mit darin befindlichen flachgefalteten Packungsmänteln in perspektivischer Darstellung,
- Fig. 4: ein zweites Ausführungsbeispiel eines oben offenen Umkartons mit darin befindlichen flachgefalteten Packungsmänteln in perspektivischer Darstellung,
- Fig. 5: ein weiteres Ausführungsbeispiel eines oben offenen Umkartons mit darin befindlichen flachgefalteten Packungsmänteln in perspektivischer Darstellung,
- Fig. 6: ein anderes Ausführungsbeispiel eines Behältnisses im Vertikalschnitt,
- Fig. 7: ein Behältnis zur Aufnahme einer Rolle Verpackungsmaterial im Vertikalschnitt und
- Fig. 8: den Ausschnitt einer Ecke eines Behältnisses mit einem innen befestigten Trägerelement.

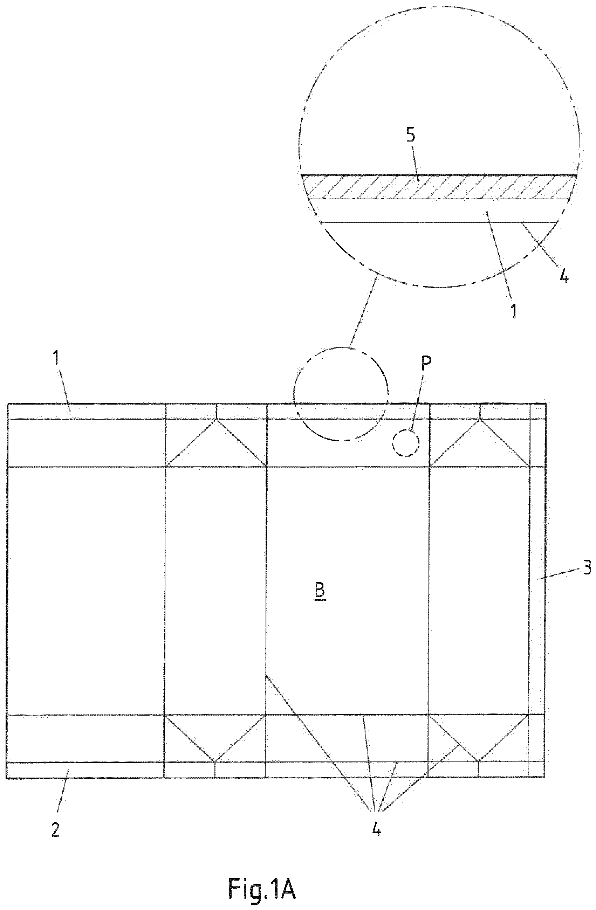

In Fig. 1A ist ein aus einem Verbundlaminat hergestellter Zuschnitt B dargestellt, der oben, unten und auf seiner rechten Seite drei Zonen 1, 2, 3 für die spätere Nahtherstellung aufweist und darüber hinaus mit einer Vielzahl von Rill- und Falzlinien 4 versehen ist, die das spätere Auffalten des fertigen Getränkekartons erleichtern und von denen nur einige in Fig. 1A mit dem Bezugszeichen 4 versehen sind. Auf der Oberseite des Zuschnitts B erkennt man eine runde Perforation P als Schwächungszone für ein aufzubringendes Ausgießelement (nicht dargestellt).

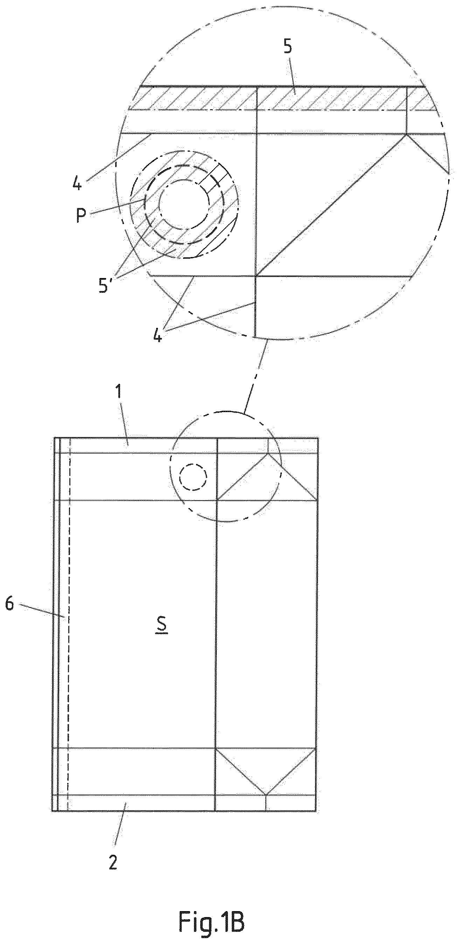

In Fig. 1B ist ein aus einem Zuschnitt B gemäß Fig. 1A hergestellter Packungsmantel S dargestellt, wobei der Zuschnitt B in seinem Nahtbereich 3 zu einer Hülse durch Siegelung einer Längsnaht 6 verbunden und zu einem Packungsmantel S flachgefaltet wurde. Das Flachfalten erfolgt aus einem einfachen Grund, da in der Regel die Herstellung der Packungsmäntel S an einem anderen Ort erfolgt als die spätere Weiterverarbeitung zu Getränkepackungen, welche in Abfüllbetrieben an unterschiedlichen Orten durchgeführt wird. Zu diesem Zweck wird eine Vielzahl flachgefalteter Packungsmäntel S aufrecht stehend in sog. ,Umkartons' 7 gepackt und zum jeweiligen Betrieb transportiert. Die Verweilzeit der Packungsmäntel S in den Umkartons 7 bis zu dessen Öffnung unmittelbar vor der Füllmaschine beträgt meist deutlich mehr als drei Tage.

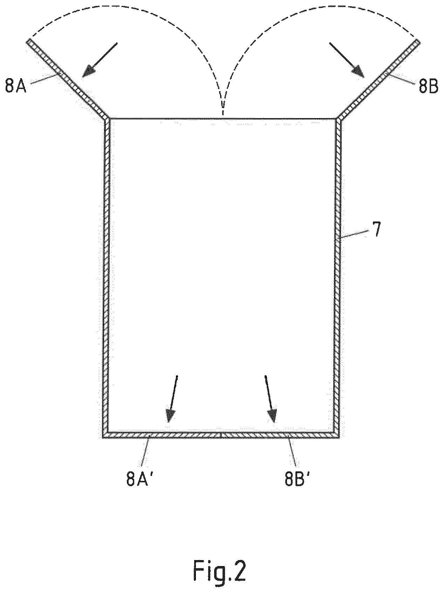

Die Erfindung hat nun erkannt, die hinsichtlich einer Kontamination problematischen offenen Packungskanten an der Unter- bzw. Oberseite der Packungsmäntel S nicht mehr direkt mit einem Entkeimungsmittel zu beaufschlagen, sondern derart, dass wenigstens ein Teil des das Entkeimungsmittel aufweisenden Behandlungsmittels auf ein Trägerelement aufgebracht wird, so dass die sich einstellende aktiv sterilisierende Atmosphäre anschließend auf die offenen Schnittkanten einwirkt und die gewünschte Sterilisation der Randbereiche 5 der Kanten bzw. der Ringbereiche 5' der Packungsmäntel S vornimmt. Als Trägerelement kann bevorzugt ein Umkarton 7 aus Wellpappe genutzt werden, bei dem die oberen und unteren Laschen 8A und 8B, 8A' und 8B' auf der Kartoninnenseite mit einem Behandlungsmittel benetzt werden und zwar im Bereich der nicht näher bezeichneten Pfeile in Fig. 2.

Auf diese Weise stellt sich im verschlossenen Umkarton 7 eine aktiv sterilisierende Atmosphäre ein, in der das Behandlungsmittel in die offenen Schnittkanten der Packungsmäntel S eindringen kann, beziehungsweise in der das im Randbereich oder einer Perforation an den offenen Schnittkanten angesiedelte Kartonmaterial dazu angeregt wird, das Behandlungsmittel zu absorbieren. Dort dringt das Entkeimungsmittel so weit (einige Millimeter) in das Kartonmaterial ein, so dass eine ausreichende Sterilisation erreicht wird.

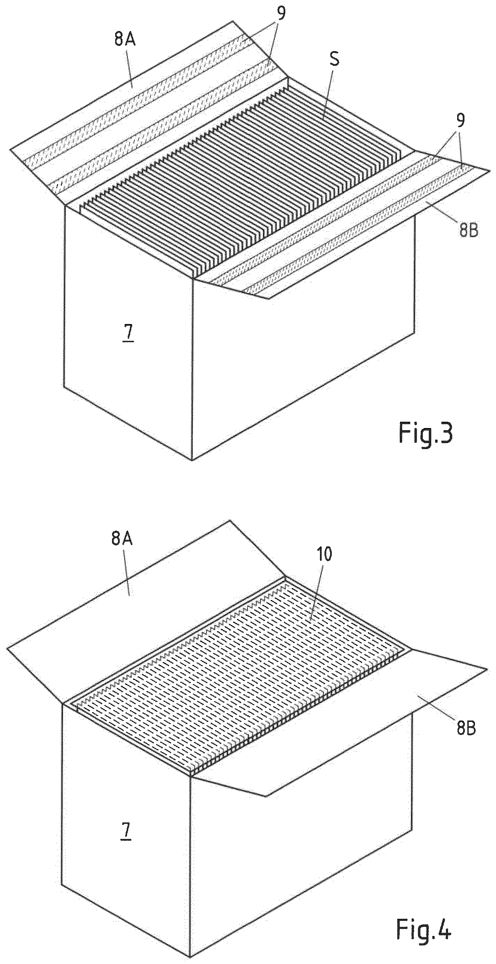

Es ist jedoch auch möglich, dass das Behandlungsmittel nicht direkt auf die Oberfläche des Umkartons 7 aufgebracht wird, sondern dort mit Hilfe eines Trägerelements oder Beladungsträgers eingebracht wird. Dazu können beispielsweise Streifen 9, wie in Fig. 3 dargestellt, aus Klebeband, Filz oder anderen geeigneten Materialien bestehen, welche auf die Innenseite des Umkartons 7 aufgeklebt werden oder im unteren Bereich des Umkartons 7 lediglich eingelegt werden können. Es hat sich gezeigt, dass eine vollflächige Benetzung nicht zwingend erforderlich ist, um eine ausreichende Sättigung der Atmosphäre mit Entkeimungsmittel zu erhalten.

Fig. 4 zeigt nun, dass es auch möglich ist, ein Einlegeblatt 10 in den Umkarton 7 (unten und/oder oben) einzulegen, wobei das Einlegeblatt 10 vollflächig oder auch nur teilweise mit der notwendigen Menge an Behandlungsmittel benetzt worden ist.

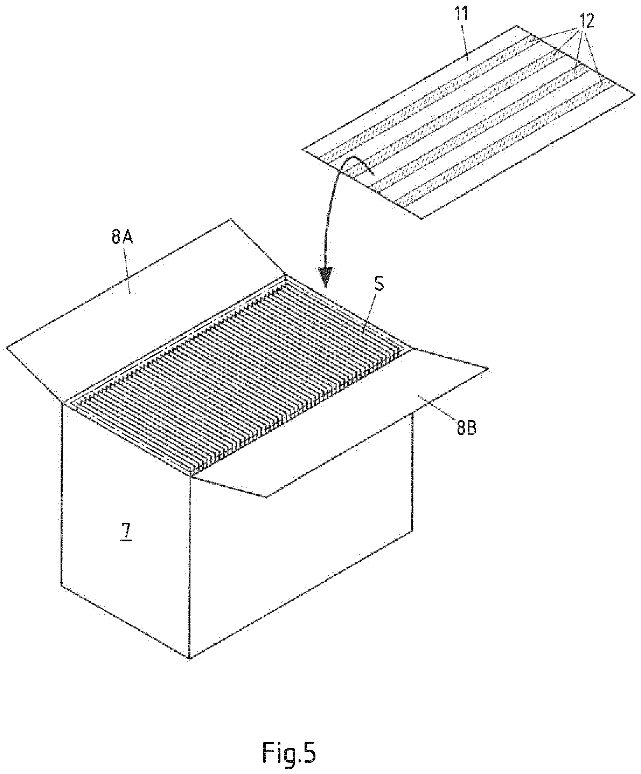

In Fig. 5 ist dargestellt, dass es auch möglich ist, ein Einlegeblatt 11 mit streifenförmigen Beladungsträgern 12 zu versehen, das dann anschließend umgekehrt auf die oberen offenen Schnittkanten der Packungsmäntel S aufgelegt wird, bevor der Umkarton 7 verschlossen wird. Selbstverständlich ist es auch möglich, vor dem Einbringen der Packungsmäntel S in den Umkarton 7 ein entsprechend vorbereitetes Einlegeblatt auf den Boden des Umkartons 7 zu legen, um auch die unteren offenen Schnittkanten der Packungsmäntel S zu entkeimen.

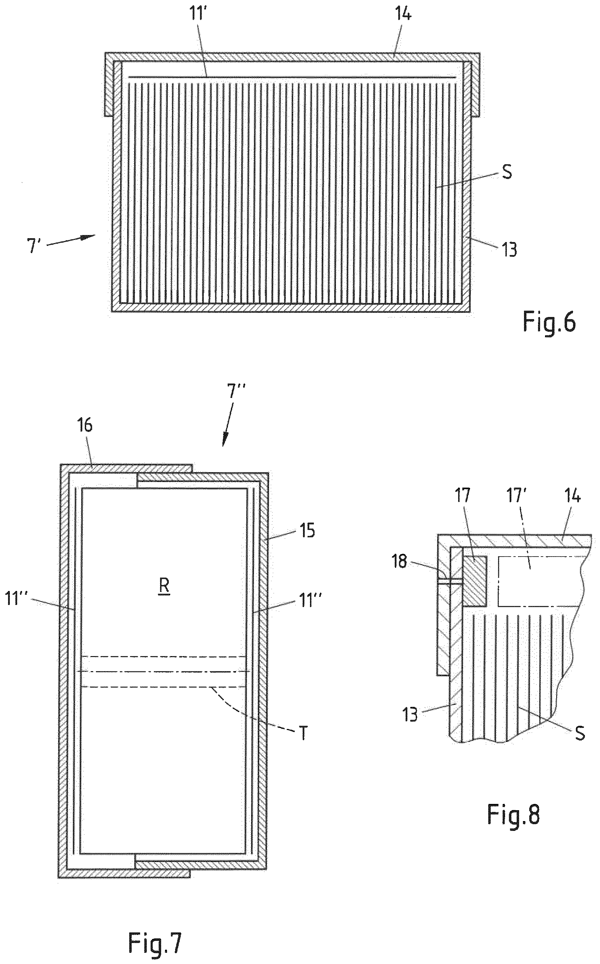

In Fig. 6 erkennt man, dass die aufrecht stehenden flachgefalteten Packungsmäntel S auch in einer ,Umverpackung' aus einem Kunststoffmaterial transportiert werden können. Man erkennt deutlich einen Kunststoffbehälter 13 im Vertikalschnitt auf dem ein entsprechender Deckel 14 aufgesetzt ist. In den Kopfraum dieser Umverpackung 7' ist ein Beladungsträger dienendes präpariertes Einlegeblatt 11' eingelegt, um die notwendige sterile Atmosphäre zu erreichen. Zum besseren Abschluss können die "offenen" Übergänge zwischen Behälter 13 und Deckel 14 auch noch mit einem Klebeband geschlossen werden.

Nicht dargestellt ist, dass es auch möglich ist, eine Mehrzahl von aufrecht stehenden und flachgefalteten Packungsmänteln nicht mit einer festen Umverpackung zu versehen, sondern beispielsweise mit einer Schrumpffolie zu umgeben, wobei vorher eine entsprechende Einlage aus Trägermaterial auf den Bereicht der offenen Schnittkanten gegeben wird, um dort die gewünschte Sterilisation durchzuführen.

In Fig. 7 ist dargestellt, dass es auch möglich ist, eine auf einer Hülse T aufgewickelte Rolle R eines Verpackungsverbundmaterials an seinen offenen Kanten mit dem erfindungsgemäßen Verfahren zu behandeln. Dies ist insbesondere dann von Vorteil, wenn die Rollenbreite genau der Packungshöhe entspricht, weil dann beide Kreisflächen der Rolle nahezu vollflächig aus offenen Schnittkanten bestehen. Dazu wird die Rolle R im dargestellten und insoweit bevorzugten Ausführungsbeispiel von einer zweiteiligen Schrumpfkappe bestehend aus einem kleineren Kappenteil 15 und einem daran angepassten größeren Kappenteil 16 umgeben. Man erkennt auch hier, dass im Bereich der offenen Schnittkanten Einlegeblätter 11" vorhanden sind, um die gewünschte sterile Atmosphäre zu schaffen. Selbstverständlich ist es auch bei der Verpackung von Rollenmaterial möglich, diese, wie zuvor beschrieben, nach dem Versehen mit Beladungsträgern 11" in Schrumpffolie zu verpacken.

In Fig. 8 ist nun am Bespiel der linken oberen Ecke des Containers aus Fig. 6 dargestellt, dass es auch möglich ist, als Beladungsträger einen etwas voluminöseren Streifen 17 aus Filzmaterial oder dergleichen einzubringen, wobei in diesem Fall eine Beladung des Streifens 17 mit Behandlungsmittel auch nach dem Verschließen des Behältnisses erfolgen kann. Dazu wird eine Wand des Behältnisses von einer Nadel durchstoßen und auf diese Weise der innenliegende Streifen 17 mit der notwendigen Menge an Behandlungsmittel getränkt, um die bereits fertig verpackten, flach gefalteten Packungsmäntel S an ihren offenen Schnittkanten ausreichend zu sterilisieren. Dazu kann der Container eine kanalvorbereitete Öffnung 18 aufweisen. Auch ist es möglich, falls notwendig, dass im Kopfraum des Containers weitere Streifen 17' als Beladungsträger vorgesehen sein können.

## Patentansprüche

1. Behältnis zur Aufnahme von Verpackungsmaterial, wobei wenigstens Teile (9, 12) der Innenseite des Behältnisses (7, 7', 7") mit einem Behandlungsmittel benetzt oder ausgestattet sind und/oder im Inneren des Behältnisses (7, 7', 7") mit einem Behandlungsmittel versehene Beladungsträger (10, 11, 11', 11", 17) vorgesehen sind und dass das Behandlungsmittel eine H₂O₂-Lösung als Entkeimungsmittel enthält,
**dadurch gekennzeichnet, dass**
das Behältnis Verpackungsmaterial in Form von einer Mehrzahl flachgefalteter Packungsmäntel (S), Zuschnitte (B) oder zu einer Rolle (R) aufgerollter Bahnware enthält,
wobei das Verpackungsmaterial aus einem Verbundwerkstoff mit wenigstens einer Lage Karton, welcher Zellstoff enthält, besteht,
und das Verpackungsmaterial jeweils offenen Schnittkanten aufweist, wobei die offenen Schnittkanten der umgebenden Atmosphäre zugängliche Zellstoffbereich bilden;
und dass das Behandlungsmittel Ethanol enthält.

2. Behältnis nach Anspruch 1,
**dadurch gekennzeichnet, dass**
die, vorzugsweise äußeren, Oberflächen des Behältnisses (7, 7', 7") wenigstens im behandelten Bereich versiegelt sind.

3. Behältnis nach Anspruch 1 oder 2,
**dadurch gekennzeichnet, dass**
als Material für das Behältnis (7) Karton, insbesondere Wellpappe, verwendet wird, so dass ein Umkarton mit schließbaren Laschen (8A, 8B, 8A', 8B') entsteht.

4. Behältnis nach Anspruch 1 oder 2,
**dadurch gekennzeichnet, dass**
als Material für das Behältnis (7', 7") Kunststoff verwendet wird.

5. Behältnis nach Anspruch 4,
**dadurch gekennzeichnet, dass**
das Behältnis (7', 7") einen Kunststoffcontainer (13) und einen Kunststoffdeckel (14) sowie wenigstens einen Beladungsträger (10, 11, 11', 11", 17) aufweist.

6. Behältnis nach Anspruch 4,
**dadurch gekennzeichnet, dass**
das Behältnis (7', 7") eine Schrumpffolie mit einem Beladungsträger (10, 11, 11', 11", 17) ist.

7. Verfahren zum Verpacken von einer Mehrzahl flachgefalteter Packungsmäntel, Zuschnitte oder zu einer Rolle aufgerollter Bahnware, jeweils aus einem Verbundwerkstoff mit wenigstens einer Lage Karton, welcher Zellstoff enthält, und mit offenen Schnittkanten, wobei die offenen Schnittkanten der umgebenden Atmosphäre zugängliche Zellstoffbereich bilden, in einem Behältnis nach einem der Ansprüche 1 bis 6,
**gekennzeichnet durch** die folgenden Schritte:
- Einbringen der flachgefalteten Packungsmäntel, Zuschnitte oder zu einer Rolle aufgerollter Bahnware, jeweils mit offenen Schnittkanten, in das Behältnis,
- Aufbringen eines Ethanol und eine H₂O₂-Lösung als Entkeimungsmittel enthaltendes Behandlungsmittels auf die Innenseite des Behältnisses und/oder auf einen Beladungsträger und Einbringen des Beladungsträgers in das Behältnis und
- Verschließen des Behältnisses.

## Claims

1. Container for receiving packaging material, in which at least parts (9,12) of the inside of the container (7, 7', 7") are wetted or provided with a treatment agent and/or load carriers (10, 11, 11', 11", 17) provided with a treatment agent are provided inside the container (7, 7', 7") and that the treatment agent contains a H₂O₂ solution as a disinfectant,
**characterised in that**
the container contains packaging material in the form of a plurality of flat-folded packaging sleeves (S), blanks (B) or web material, rolled up into a roll (R), wherein the packaging material consists of a composite material comprising at least one cardboard layer which contains cellulose, and the packaging material respectively has open cut edges, wherein the open cut edges form cellulose zones accessible to the surrounding atmosphere; and **in that** the treatment agent contains ethanol.

2. Container according to claim 1,
**characterised in that**
the, preferably outer, surfaces of the container (7, 7', 7") are sealed at least in the treated region.

3. Container according to claim 1 or 2,
**characterised in that**
cardboard, particularly corrugated board, is used as material for the container (7), thereby producing a covering box with closable flaps (8A, 8B, 8A', 8B').

4. Container according to any one of claims 1 or 2,
**characterised in that**
plastics is used as material for the container (7', 7").

5. Container according to claim 4,
**characterised in that**
the container (7', 7") comprises a plastics container (13) and a plastics lid (14) as well as at least one load carrier (10, 11, 11', 11", 17).

6. Container according to claim 4,
**characterised in that**
the container (7', 7") is a shrink film with load carriers (10, 11, 11', 11", 17).

7. Method for packaging a plurality of flat-folded packaging sleeves, blanks or web material, rolled up into a roll, respectively consisting of a composite material comprising at least one cardboard layer which contains cellulose, and further comprising open cut edges, wherein the open cut edges form cellulose zones accessible to the surrounding atmosphere, in a container according to any one of claims 1 to 6,
**characterised by** the following steps:
- the flat-folded packaging sleeves, the blanks or the web material, rolled up into a roll, in each case provided with open cut edges, are introduced into the container,
- a treatment agent containing ethanol and a H₂O₂ solution is applied as a disinfectant to the inside of the container and/or to a load carrier, and the load carrier is inserted into the container; and
- closing of the container.

## Revendications

1. Récipient destiné à la réception d'un matériau d'emballage, dans lequel au moins des parties (9, 12) du côté interne du récipient (7, 7', 7") sont humectées avec un moyen de traitement ou équipées de ce dernier et/ou à l'intérieur du récipient (7, 7', 7") sont prévus des supports de chargement (10, 11, 11', 11", 17) munis d'un moyen de traitement et en ce que le moyen de traitement contient une solution de H₂O₂ à titre de moyen de désinfection,
**caractérisé en ce que**
le récipient contient un matériau d'emballage sous la forme d'une multitude d'enveloppes d'emballage mises à plat (S), de découpes (B) ou d'un produit en bande enroulé pour obtenir un rouleau (R), dans lequel le matériau d'emballage est constitué d'un matériau composite comprenant au moins une couche de carton qui contient de la cellulose, et le matériau d'emballage présente respectivement des arêtes de coupe ouvertes, dans lequel les arêtes de coupe ouvertes forment des zones de cellulose accessibles à l'atmosphère environnante ; et **en ce que** le moyen de traitement contient de l'éthanol.

2. Récipient selon la revendication 1,
**caractérisé en ce que**
les surfaces, de préférence externes, du récipient (7, 7', 7") sont scellées, au moins dans la zone traitée.

3. Récipient selon la revendication 1 ou 2,
**caractérisé en ce que**,
à titre de matériau pour le récipient (7), on utilise du carton, en particulier du carton ondulé, d'une manière telle que l'on obtient un emballage extérieur comprenant des pattes qui peuvent être fermées (8A, 8B, 8A', 8B').

4. Récipient selon la revendication 1 ou 2,
**caractérisé en ce que**,
à titre de matériau pour le récipient (7', 7"), on utilise une matière synthétique.

5. Récipient selon la revendication 4,
**caractérisé en ce que**
le récipient (7', 7") présente un conteneur en matière synthétique (13) et un couvercle en matière synthétique (14), de même qu'au moins un support de chargement (10,11,11', 11", 17).

6. Récipient selon la revendication 4,
**caractérisé en ce que**
le récipient (7', 7") est un film rétractable qui comprend un support de chargement (10, 11, 11', 11", 17).

7. Procédé destiné au conditionnement d'une multitude d'enveloppes d'emballage mises à plat, de découpes ou d'un produit en bande enroulé pour obtenir un rouleau, respectivement constitués d'un matériau composite comprenant au moins une couche de carton qui contient de la cellulose, et comprenant des arêtes de coupe ouvertes, dans lequel les arêtes de coupe ouvertes forment des zones de cellulose accessibles à l'atmosphère environnante, dans un récipient selon l'une quelconque des revendications 1 à 6, **caractérisé par** les étapes suivantes :
- on introduit les enveloppes d'emballage mises à plat, les découpes ou le produit en bande enroulé pour obtenir un rouleau, respectivement munis d'arêtes de coupe ouvertes, dans le récipient ;
- on applique un moyen de traitement qui contient de l'éthanol et une solution de H₂O₂ à titre de moyen de désinfection sur le côté interne du récipient et/ou sur un support de chargement et on introduit le support de chargement dans le récipient ; et
- on ferme le récipient.
